# EUROPEAN PATENT APPLICATION

(11) **EP 1 541 328 A1**
(43) Date of publication of application: **15.06.2005**
(21) Application number: 02752958.5
(22) Date of filing: 19.07.2002
(51) Int. Cl.: B30B 11/28, B01J 2/20, C10L 5/44, C12P 7/08

(54) **A PROCESS AND A DEVICE FOR SHAPING THE LOOSEN MATERIAL OF RAW VEGETABLE MATTER**

(71) Applicant: Che, Zhanbin, Haidian District, Beijing 100080 (CN); Ni, Weidou, Beijing 100084 (CN)
(72) Inventor: Che, Zhanbin, Haidian District, Beijing 100080 (CN); Ni, Weidou, Beijing 100084 (CN)
(74) Representative: Bogsch, Adam, Dipl.-Ing.
(86) International application number: PCT/CN2002/000506
(87) International publication number: WO 2004/009340

(57) **Abstract**

A molding method for moldable bio-substance materials comprises crushing the bio-substance materials into an incompact state, and then extruding and molding the same. There is at least a wedged extruding cavity is formed between the extruding head and the extruding surface of the molding die. The movement between the extruding head and the extruding surface of molding die is a differential speed movement, and the particles of the materials are extruded between these two surfaces and are grinded, twisted up, stretched, flaked, and then extruded to the small end of the wedged extruding cavity, and further into the molding cavity of molding die to be molded therein. The molding method and equipment of this invention can give the molded product certain strength and water-fastness without using any chemical binders. It is convenient for storing the product and keeping the product in a functional state, and thus it minimizes the cost for fabrication, transportation and storage.

## Description

### FIELD OF THE INVENTION

The present invention relates to a method of molding loose bio-substance materials. It possesses the ability to mold the loose bio-substance materials into various required shapes without using any chemical binder, while the molded product will have sufficient linking strength.

### BACKGROUND OF THE INVENTION:

The moldable bio-substance materials of the present invention is made from raw materials including the solid debris from wood processing, straw of crop, herbage plant and bush, which are treated into a loose and spongy state and mould into useful materials of desired shapes. These raw materials of bio-substance are composed of wastes from natural plants and thus are characterized by their low-cost, redeemable and rich resources.

At present, the usage of these bio-substance materials mainly includes the following areas:
1. Loose materials are molded into artificial board or other shaped materials, such as fiberboard, shaving board, etc. for furniture making and decoration projects. The traditional fabrication technology for making these kinds of artificial boards includes adding and mixing large amount of chemical binders and dipping materials into the loose materials, and then molding under heat and pressure. Therefore, during fabricating, storing and using of such artificial boards, large amount of dissociated formaldehyde will be released causing pollution of the environment.
2. Loose bio-substance materials are molded into shapes of stick or pellet for using as combustive materials to substitute fossil fuel. The combustion emissions thereof do not have any deleterious gases, such as sulfur dioxide and nitrogen oxide. Therefore, it is the best substitute combustive material for fossil fuel. However, the volume of unshaped loose bio-substance material is so bulky for transportation and storage, and thus the cost of using such materials is very high. It is then necessary to mould or compact such loose bio-substance materials, and substantially reduce its volume, and improve combustion efficiency per unit volume. In this way, such loose bio-substance materials could have practical value.

However, as a bio-substance combustion material, it is very important to keep its original emission characteristics; namely, no chemical binder and dipping material should be added therein while it makes very difficult to mould such loose bio-substance material. In this regard, scientists from allover the world have found the basic formation or molding principle of such bio-substance materials through extensive researches. At present, the widely-approved molding principle of such bio-substance materials is that in addition to cellulose and semi cellulose, botanic cell also contains lignin which is a macromolecule compound with the property of aroma family, the mono structure of which is stereo phenylalkyl. Lignin is non-crystal, and most of separated lignin do not have fixed fusion point but soften point. The binding force increases when the temperature falls within 70~110°C. The lignin will be softened and liquefied when the temperature is 200~300°C. If a certain pressure is applied at this time to bring the fibrin close and to bind adjacent particles together, it will become solid and be molded as required. This is the way to mould the bio-substance materials without any binders.

Based on the above mentioned principle, the condition for molding the bio-substance materials depends on the properties of softening and liquidation of the lignin. Firstly, there are some requirements for the type, particle size and water content of the bio-substance. Usually, it is required that the particle size of the bio-substance material should be below 10mm and the water content should be less than 10%. However, the water content of the bio-substance usually falls between 20%~40%, and therefore it is necessary to dry the materials before molding. In view of the above mentioned requirements for raw bio-substance material, the conventional molding process of such bio-substance is: raw bio-substance material → crushing → drying → extruding →packing.

It is well known that the extruding process is the key of solidifying technology for the above mentioned molding process. During extruding process, the most important factors are pressure and temperature. Lignin will not be softened and melted to bind particles together without a certain temperature and pressure.

It is proved by extensive reference searches that screw molding machine is the most popular type for transportation and extrusion, material is continuously extruded into a die to be molded at higher heating temperature. Usually, the set heating temperature should meet the required temperature for lignin softening and liquefying, namely 240~260°C. And then the materials are cooled down and solidify. This process is to let the lignin in bio-substance stay melting, which will be beneficial for the binding between cellulose and semi cellulose. However, during the high temperature heating process in the die, when the bio-substance material has high water content, the surface of the product will be cracked and even fulminated at the worst situation, if the vapor produced from the heating process can not be discharged smoothly. Therefore, this process requires that drying must be conducted after the bio-substance is crushed, and before the particles can be sent to the molding machine for extrusion. During extruding process, electric heating system is used to generate high temperature under which the lignin will be forced out of the bio-substance and help binding.

It has been demonstrated during long term application that the above mentioned traditional process has the following unconquerable defects:
**I.** High energy consumption due to following reasons:
   (1) Bio-substance material must be dried before molding to reduce its water content to less than 10%. It will need to consume a lot of energy when used in industrial large scale productions;
   (2) When bio-substance material is dried until its water content falls to less than 10%, the friction will be greatly increased during extruding process, which will cause higher energy consumption per unit; and
   (3) During solidifying process, it is necessary to heat the material which also requires large energy consumption.

   It has been proved by practice that the reason why bio-substance molding process cannot be popularized in large scale so far is because of its high energy consumption and therefore high production cost.
**II.** Since the existing method for bio-substance molding requires strong energy support, the production must be applied under high voltage power and in concentrative production mode which requires the raw bio-substance material be transported to a fixed production site. It is well known that the radius for collection and transportation of incompact bio-substance is very large, thus the cost for transportation and storage of bio-substance is very high. This is another important reason for the high cost of bio-substance molding.
**III** It has been confirmed by practice that the products made by the above mentioned method have the defect of high brittleness and low water-fastness, its linking strength will be much reduced when it is damp, which makes it easy to be crashed. For example, when put into water, the bio-substance combustion products made by the above mentioned method will become powder in a few minutes. Therefore, the conditions of storage for such product are very strict. Even though the cause of such defect is not very clear, it is considered by the inventor that the above molding method is based on the high viscosity property of lignin in its melting state. Lignin has high viscosity when it is melted, but shows high brittleness and low water-fastness after cooling down. Therefore, lignin will cause the low water-fastness of the product when it is used as the main binder for binding fibrin. This is also the reason why it is almost impossible to make artificial board, such as fiberboard and shaving board without binder using the above mentioned method.
**IV** At present, most molding processing methods include screw extruding or hydraulic pressing. The feature of these methods is to apply positive and continuous pressure on the material which will thus be molded. Some Chinese scientists have studied the particles deformation and binding formation during compression and molding. A model of microcosmic combination during the molding process was established and the study showed that during molding process, part of particles will continually enter the interstice between particles when the pressure is low at beginning, and the relative position between particles is continually renewed. After all the large interstices being occupied by entering particles, the only way to fill in around interstice under further increased pressure is to rely on the deformation of particle itself. At this moment, the particle is stretched in the plane which is vertical to the direction of the maximum main stress. And the particles will be linked together under further pressure, when the particle is stretched to such point that the adjacent two particles could physically contact to each other.
   It has been proved by practice that the products made through the above mentioned method has low linking strength. After a lot of study for the force conduction of biosubstance material, the inventor has found that the force conduction of the incompact bio-substance material is poor. Therefore, after all the large interstices being occupied by entering particles, the deformation of particle itself is small even through further increased pressure and it is difficult to make the particle fully stretch. This is the reason why idealistic linking state is difficult to reach, and thus is an important key, however, overlooked technology for the existing molding method.
**V.** Usually, screw extrusion molding machine is used for bio-substance product molding. It is complicated in structure and low in production efficiency. Especially when water content of bio-substance drops to less than 10%, the erosion of the screw is serious at high temperature and dry friction conditions. The average operation life of the screw is only about 60 to 80 hours.

To summarize, in order to effectively utilize bio-substance pellet without causing pollution, the following important problems must be solved:
(1) Molded product must have necessary linking strength and water-fastness; (2) Production cost should be greatly lowered; (3) Chemical addition for molded product should be greatly reduced, in order to lower environmental pollution during production and utilization of molded bio-substance product.

### SUMMARY OF THE INVENTION

An object of this invention is to provide a molding method for bio-substance material, the product of which will have a certain linking strength and water-fastness to keep its normal state for utilization and easy for storage without using any chemical binder.

Another object of this invention is to provide a molding method for bio-substance material, which will minimize the cost for its product manufacture, transportation and storage.

The molding method provided in this invention is: a type of molding method for moldable bio-substance material, which will be crushed into incompact particles, and then extruded into the required shape. In this system, at least, a wedged extruding cavity will be formed between extruding head and molding die. Due to differential speed, relative movement between extruding head and molding die will occur and particles inside will be grinded, twisted up and stretched. The particles will then be shoved into the small end of extruding cavity at the same time and finally extruded into molding cavity in the molding die to be molded.

This invention is a molding mechanism for moldable bio-substance material, which includes at least an extruding head and a molding die driven by power, the characteristics is that there is at least a wedged extruding cavity between extruding head and molding die, the big end of wedge extruding cavity has inlet for the bio-substance material. Particles of bio-substance enter into the wedge extruding cavity, will be grinded, twisted up and stretched by the action of relative movement between extruding head and extruding surface of molding die. And particles will enter molding cavity in the molding die after becoming flake-shaped.

The main features of the molding method of this invention include following aspects:
**A.** Water content of bio-substance material used as feedstock for the process of this invention should be greater than 16%, water content of 20%~50% is usually preferred. 20%~50% is actually the natural water content of bio-substance. In this case, drying the bio-substance becomes unnecessary before processing, which saves energy for drying. High water content can soften the particles for easy deformation. Therefore, under same extruding condition, the deformation of particles will augment accordingly, which will also greatly reduce the extruding pressure and energy consumption.
**B.** This invention is to extrude and mould bio-substance material at normal temperature, which means high temperature for heating is not necessary during the molding process. Thus the energy for heating bio-substance material could be saved. Most importantly, this method only use the action of extruding press and shearing force and friction between the particles when bio-substance material is under extruding condition, which will increase its temperature to 70~100°C to soften lignin. The softening lignin has a certain binding force but will not be melted at the temperature of 70~100°C. Therefore, the product produced by this method will have better plasticity and water-fastness. It has been proved through many tests by this inventor that the product produced by this method can keep its original shape even after soaking in water for more than 40 hours, and it will keep original application function after being dried. As for the product produced by other existing methods, it will be in a state of powder when soaking in water for 10 minutes.
**C.** During extruding and molding process of the inventive method, a shearing force will be applied on the material before the material enters molding die. Particles in the extruding cavity are then twisted up, stretched and flaked by the action of shearing force. Flaky material enters molding die in the folding state to be further extruded, while the volume of extruding cavity getting smaller. During this process, not only the density between layers is getting greater, but also the flaky particles are deformed by the action of positive extruding force. After deformation, part of the particles enter the gap between flaky particles to mesh into each other, which forms the specific structure model of the product suggested by this inventive method and better mechanics feature than the products produced by other existing methods. The product produced by this inventive method has a special mechanics feature of greater radial and axial tensile strength, thus the product will have better linking strength. Some Chinese scientist gave an explanation for this mechanics feature as it is because of the difference of linking between radial particles and axial particles. In radial direction, particles mesh to link together; to break such linkage, a larger action force is required either to separate the particles at the linking position or to destroy the particle itself. In axial direction, particles not only joint to link together, but also mesh to link together.
   This inventive method has effectively solved the problem of poor force transfer for bio-substance material, by changing deformation law for the bio-substance material molding, firstly stretching for deformation and then extruding for molding. It has been proved by experiments that the molded product produced by this method has excellent linking strength. Thus the usage of chemical binder can be minimized. And the product could even be use for furniture fabrication without any binder.
**D.** Since extensive heating is not required in this inventive method, large power support will not be needed by the molding equipment (pelletizer). In this case, if needed, this invention can be designed as moving equipment carried by truck while using truck engine's power or other tolling equipment. Thus this inventive method can be used onsite of the field and make it unnecessary for the transportation of bio-substance material to centralized production site. By applying this method, transportation cost and production cost could be greatly reduced, which makes it practical to utilize bio-substance material with low cost.

Bio-substance combustion material produced by this inventive method is a potential substitution for the fossil fuel energy, due to its low transportation and production cost, possibility to be produced and sold locally, and its non-polluted combustive emissions.

It has been proved by a many tests that the product made from incompact bio-substance material has the following advantages: 1) the product has higher linking strength without using any chemical binder, thus completely avoiding environmental pollution; 2) the product has greatly reduced moisture absorption and much better water-fastness, which makes it suitable to be utilized or stored in a moist environment; 3) the production cost of this product is greatly reduced, which could make popular of the utilization for bio-substance material.

### BRIEF DESCRIPTION OF DRAWINGS

Figure 1 is a sketch of working and structural principle for implementation example 1 of this invention;
Figure 2 illustrates the working and structural principle for implementation example 2 of this invention;
Figure 3 shows the structure of extruding head in Figure 2;
Figure 4 illustrates the working and structural principle for implementation example 3 of this invention;
Figure 5 is another kind of working and structural principle for implementation example 3 of this invention;
Figure 6 shows the working and structural principle for implementation example 4 of this invention;
Figure 7 illustrates another kind of working and structural principle for implementation example 4 of this invention;
Figure 8 shows the working principle for implementation example 5 of this invention;
Figure 9 shows the working and structural principle for implementation example 6 of this invention;
Figure 10 shows the working and structural principle for implementation example 7 of this invention;
Figure 11 is illustration of working and structural principle for implementation example 9 of this invention;
Figure 12 shows the working and structure principle for implementation example 10 of this invention;
Figure 13 illustrates the working and structural principle for implementation example 10 of this invention;
Figure 14 is a sketch of structural principle for implementation example 10 of this invention;
Figure 15 is sketch of working and structural principle for implementation example 11 of this invention;
Figure 16 shows the working and structural principle for implementation example12 of this invention; and
Figure 17 shows the working and structural principle for implementation example 13 of this invention

### DETAILED DESCRIPTION OF THE INVENTION

This invention provided a molding method for plastic bio-substance, feedstock in this invention is bio-substance material which is made from raw materials including plant, wood processing offal, crop straw, herbage plant and frutex, by crushing them into incompact plastic state. It has been proved in both theory and practice of bio-substance molding that the smaller the crushed particle sizes of bio-substance are, the more effective the molding process is. Therefore, the molding method of this invention, the required size of bio-substance will be same as that of the existing technology, usually, the crushed size distribution of bio-substance should be less than 10mm, less than 2~4mm of crushed size is preferred for more effective molding.

In addition, in this inventive method, crushed bio-substance material can be extruded to directly mould the product, without strict requirement for water content of the bio-substance material. Usually, water content of bio-substance without drying treatment is between 16%~50%. It has been proved by the inventor's experiments that water content range of 6%~50% will not affect the molding if using this inventive method. Therefore, it is not necessary for the dry treatment of the bio-substance material before molding process Bio-substance can then be processed directly, which will save large amount of energy for drying treatment. It has also been proved by the inventor's experiments that the particles of bio-substance will be softened and easy for deformation because of the increase of its water content during molding process, also the particles deformation can be augmented and extruding press can be much reduced under the same extruding condition. Thus energy consumption is greatly reduced.

This invention is mainly for the improvement of molding method for bio-substance material, which is to form at least a wedged extruding cavity between extruding head and molding die. The extruding surface between extruding head and molding die conduct a relative movement due to the differential speed, where the bio-substance particles extruded will be twisted up, stretched and flaked. At the same time, the particles are extruded into the small end of the wedged extruding cavity and finally are molded in the molding die cavity.

The most important molding feature of this invention is: extruding and molding are conducted at normal temperature, in another word, there is no need to use high temperature for heating, and energy consumption for heating can be saved. Another important feature of this method is that only the action of extruding press, shearing force and friction between the particles are used in the process, when bio-substance material is under extruding condition. This will increase its temperature to 70~100°C to soften lignin, which will let softening lignin have a certain binding force but not melt. Therefore, using this method, the product will have better plasticity and water-fastness. It has been proved by this inventor through many tests that the product produced by this method can maintain its original shape even after being soaked in water for more than 40 hours. And it will still keep original application function after being dried. As for the product produced from other existing methods, it will be in a state of powder after being in water for 10 minutes.

The mechanism of this invention is: during extruding and molding process of this inventive method, before being put into the molding die, the material will be extruded in a wedged extruding cavity, which is formed between extruding head and extruding surface of the molding die. When a relative movement between extruding head and extruding surface of molding die occurs, a shearing force is applied to the material extruded in a wedged extruding cavity along with the relative movement of the extruding head and the extruding surface of molding die. Particles in the extruding cavity are then be twisted up, stretched and flaked by the shearing force. Along with two surfaces with relative movement in the extruding cavity, the particles are extruded, forced to the small end of extruding cavity, continuously twisted up, stretched and extruded while the volume of extruding cavity decreases. Finally, the material will become flaky and enter molding die layer upon layer to be further extruded in a folding state. The density between layers will increase after being further extruded, which will form the same structure model as that of the existing molding method. After entering molding die, the flaky particles will be deformed by the positive extruding force, and part of the deformed particles will enter the gap between the flaky particles and mesh to each other. This will form the special structure model of the product made by this inventive method. Such product has greater tensile strength and better mechanics features than the products produced by other existing methods. The special mechanic features include greater radial and axial tensile strength which gives the product better linking strength. An explanation for theses mechanic features given by some Chinese scientist showed that it is because of the difference of linking form between radial particles and axial particles. In radial direction, particles mesh to link together; to break its linkage, a larger action force is needed either to separate the particle from linking section or to destroy the particle itself. In axial direction, particles not only joint to link together, but also mesh to link together.

This inventive method has effectively solved the problem of poor force transfer for bio-substance material by changing deformation law for the bio-substance material molding, in which materials are firstly stretched for deformation and then extruded for molding. It have been proved by experiments that the molding product produced by this method has excellent linking strength, which can minimize the use of chemical binder, or even make it possible to use such product in furniture fabrication and decoration boards without any binder.

The following are examples of implementation, which can illustrate the effectiveness of this invention in detail:

### Example 1

The method used for this example is suitable for the molding process of a bio-substance combustion material. Before the molding process, the bio-substance material which will be used for molding process should be in an incompact state, thus if raw bio-substance material is not in an incompact state, such as straw of crop and frutex, etc., it should be crushed into an incompact state. However, for the raw bio-substance material which is in an incompact state, such as saw dust, the material's particle size will determine whether it should be crushed.

In this example, incompact bio-substance material can be sent directly to the wedged extruding cavity and extruded and molded. A wedged extruding cavity will be formed between the extruding head and extruding surface of molding die. In this example of implementation, the above mentioned wedged extruding cavity can be formed between the end surface of the extruding head and molding die, and the extruded material will enter into extruding cavity from the large end of extruding cavity.

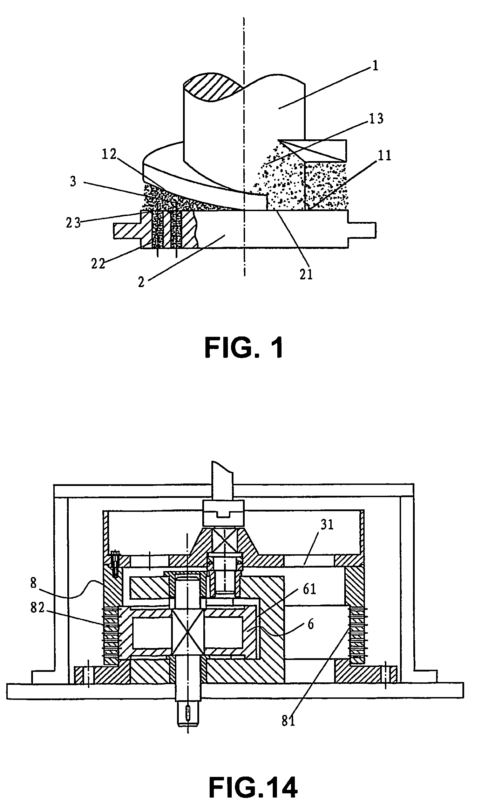

By utilizing the molding method in this example of implementation, the molding mechanism as shown in Figure 1 can be adopted. Figure 1 shows that the extruding head is in column configuration. The extruding head 1 has an end surface 11, which will at least form a ringed sloping surface 12. The molding die 2 of this invention has a plane extruding surface 21. The end surface 11 of the extruding head 1 is arranged relative to the end surface 21 of this molding die 2, which forms a wedged extruding cavity 3 between the ringed sloping surface 12 of the extruding head 1 and the extruding surface 21 of the molding die 2. At the high end of the extruding head's 1 sloping surface 12, there is a radial opening, which forms an inlet of material 13; bio-substance material will enter into wedged extruding cavity 3 from the inlet 13 to be extruded.

The coordination clearance of the end surface 11 of the extruding head 1 and the extruding surface 21 of the molding die 2 is less than about 3mm, but the coordination without clearance is the best. The radial height of the small end of the wedged extruding cavity 3 should be less than 3mm so as to form a bigger extruding force.

This wedged extruding cavity 3 is arranged along the moving direction of the extruding head from the small end to the large end. When the extruding head 1 moves relative to the molding die's 2 extruding surface 21, a shearing force will be applied to the bio-substance material which enters the extruding cavity 3 during its movement, and then the material will be twisted, stretched, and flaked by the action of this shearing force. Furthermore, the friction of the material in the extruding cavity 3 will produce heat by the action of shearing force, which will raise its temperature to 70-110°C. At the same time, the material, at a temperature of 70-110°C, will move with the extruding head to be extruded to the small end of the wedged extruding cavity 3 and then into the molding die's 2 molding cavity 22.

The extruding head of the extruding mechanism for this example of implementation can be a cylinder. The molding die 2 has a circular extruding end surface 21, and there is a molding cavity 22 which is arranged with the extruding end surface 21 at an angle. The directional section 23 can be arranged for the extruding cavity 3, which is arranged with the extruding surface of the molding die at an angle. For the processing of bio-substance combustion material, the cavity body of this molding cavity 22 can be a cylinder, which will be arranged at the molding die 2 along the extruding surface 21. In this way, every molding action can produce multi-claviform combustion material. The material in the extruding cavity 3 continually enters into the molding cavity 22 by the action of shearing force to be molded into claviform state. Under the extruding action of the latter entering material, the previous material which enters into the molding cavity 22 will be continually extruded, and become more dense. At this point, a part of the bio-substance particles which have been grinded, twisted, and flaked in the extruding cavity will be deformed. After deformation, they will enter into the gap of the other part of the flaked particles to further mesh together. Consequently, based on this invention, a special structural mold and mechanical features are given to the molded product, which is better than that based on existing technology. Finally, the molded product is extruded from the outlet end of molding cavity.

The extruding mechanism in this example of implementation is arranged in an inner cavity of extruding equipment, which is a cylinder inner cavity; the extruding head 1 can be driven by a power drive, which will be turning along a vertical axis. The molding die is a static design and a relative slipping movement mode will occur between the end surface 11, the slope surface 12, and the molding die's 2 extruding surface 21 which forms a relative movement due to differential speed between the extruding head 1 and the extruding surface 21 of the molding die 2. During the process of slipping of plane due to differential speed movement, bio-substance particles extruded between these two surfaces will be ground, twisted, and flaked. As this occurs, the movement direction will force the bio-substance material to be extruded into the small end of the wedged extruding cavity 3, and finally the flake particles will be extruded into molding cavity 22 of the molding die.

In this example of implementation, when there is no clearance for the coordination between the end surface 1 of the extruding head 1 and the extruding surface 21 of the molding die 2, the bio-substance material in the extruding cavity which has been ground, twisted, and flaked will discontinuously (not continuously) enter into the molding cavity 22, thus, the layer-built flake material in its folding state will be extruded into molding die 22. It has been proven by experiments that this molding method will be much more propitious in overcoming the objection of poor force conductivity for incompact bio-substance material, which makes the molded product have a much more reasonable structural mold of bio-substance material.

### Example 2

The molding method, molding principle and molding mechanism in this example of implementation are the same as that of example 1; therefore, unnecessary details will not be given.

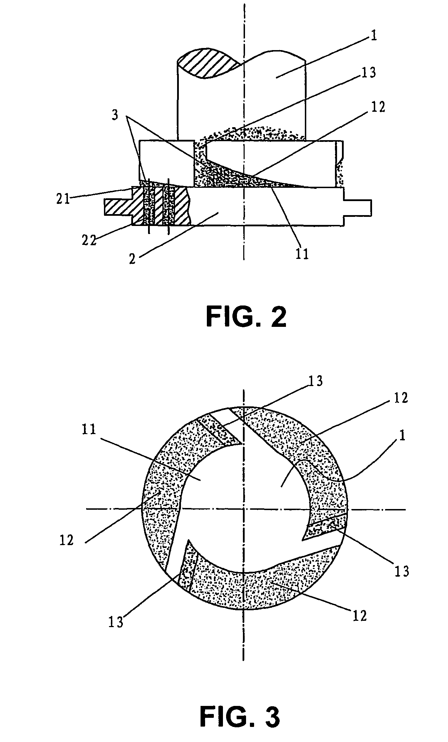

As shown in Figure 2 and Figure 3, the difference between this example and example 1 is that there is no more than two ringed sloping surfaces 12 at the end surface 11 of the extruding head 1. This ringed sloping surface 12 and extruding plane surface 21 of the molding die 2 form the wedged extruding cavity 3, the number of which will be greater than two. These wedged extruding cavities 3 will be arranged from large end to small end in order to link together.

The movement mode of this example of implementation can be the same as that adopted by example 1 of implementation, namely the extruding head can be driven by a power drive, which will be turning along a vertical axis. Its turning direction is along the extruding cavity 3 in the same direction from the small end to the large end, which will tend to make bio-substance material in the extruding cavity 3 move towards the small end of the extruding cavity. The molding die can be a static design, a relative slipping movement mode will occur between the end surface 11, the slope surface 12, and the extruding surface 21 of the molding die 2., which forms a relative movement due to differential speed between the extruding head 1 and the extruding surface 21 of the molding die 2.

The molding method and molding mechanism in this example of implementation are also suitable for mold processing of bio-substance material. Because this example of implementation uses a multi-wedged extruding cavity 3, it can improve processing efficiency.

### Example 3

The molding method, molding principle, and molding mechanism in this example of implementation are the same as that of example 1 and 2; therefore, unnecessary details will not be omitted.

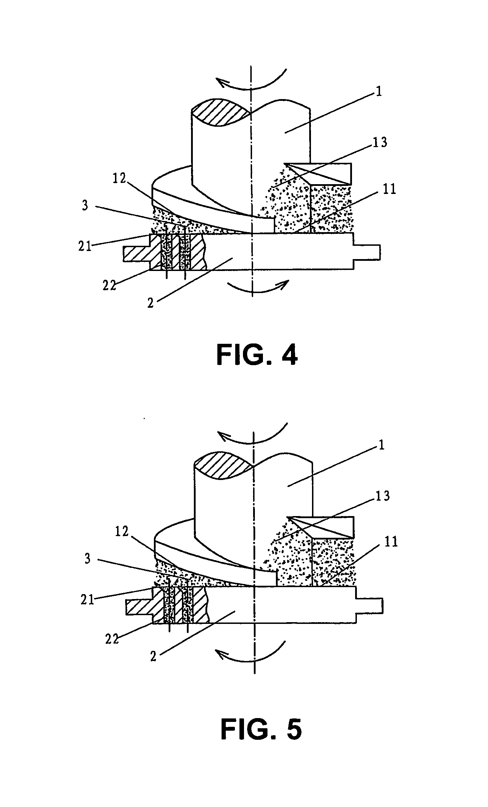

As shown in Figure 4, the difference between this example and example 2 is that the movement mode of the extruding head 1 relative to the molding die of this example is different in that the molding die can be driven by a power drive, which will be turning along a vertical axis. Its turning direction is from the small end to the large end along the extruding cavity, which will tend to make bio-substance material in the extruding cavity 3 move towards the small end direction of the extruding cavity. The extruding head 1 can be a static design, causing a relative slipping movement mode to occur between the end surface 11, the slope surface of the extruding surface, 21 and the molding die 2. This forms a relative movement due to differential speed between the extruding head 1 and the extruding surface 21 of the molding die 2.

As shown in Figure 5, in this example of implementation, the movement mode of the extruding head 1 relative to the molding die can be as follows. A power drive is turning both the extruding head 1 and the molding die 2 in the same direction from the small to the large end. However, their turning speeds differ in that N_{extruding} ≠ N_{molding}, which forms a relative movement due to differential speed. The molding effect will be better when N_{extruding} > N_{molding}.

In this example of implementation, the movement mode of the extruding head 1 relative to the molding die can be also as follows. A power drive is turning both the extruding head 1 and the molding die 2. The turning direction of the extruding head 1 is from the small end to the large end, but the turning direction of the molding die 2 can be contrary to that of extruding head 1. For this relative movement mode, both turning speeds can be either the same or different.

### Example 4

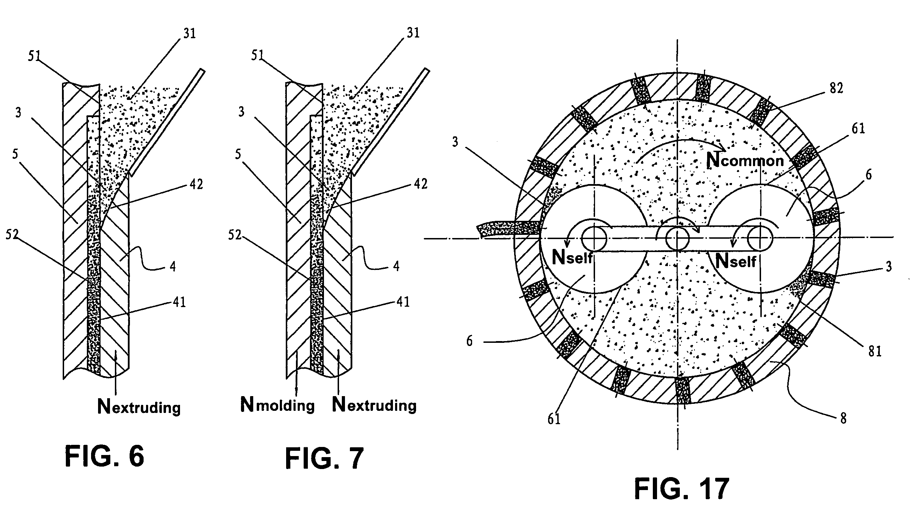

The molding principle and structure principle of the molding mechanism are shown in Figure 6. The extruding head 4 in this example of implementation is a board configuration. This extruding head 4 has an end surface 41 with board configuration, which forms at least an approximate linear slope surface 42. In this example of implementation, the molding die 5 has a plane extruding surface 51. The arrangement of the end surface 41 of the extruding head 4 is relative to the end surface 51 of the molding die 5, which can form a wedged extruding cavity 3 between the approximate linear slope surface 42 of the extruding head 4 and the extruding surface 51 of the molding die 5. The large end of this extruding cavity 3 is higher than small end. The large end will be connected directly with the material inlet 31, and incompact material can enter into the wedged extruding cavity 3 from the inlet 31.

The coordination clearance between the end surface 41 of the extruding head 4 and the extruding surface 51 of the molding die 5 is less than 3mm. The best coordination clearance is no clearance, which will make radial height of the small end for the wedged extruding cavity 3 smaller than 3mm so as to form a larger extruding force.

The arrangement of this extruding head 4 will be from small to large along the direction of its movement, when the extruding head 4 relative to the extruding surface 51 of the molding die 5 moves, the material which enters into extruding cavity 3 is applied to a shearing force during the relative movement between the extruding head 4 and the molding die 5, which is ground, twisted, and flaked by the action of shearing force. The friction of the material in the extruding cavity 3 will produce heat by the action of the shearing force, which will increase its temperature to approximately 70-110°C, at the same time, the material, now at a temperature of 70~110°C, will move with the extruding head to be extruded to the small end of the wedged extruding cavity 3 then into the molding cavity 52 of the molding die 5.

In this example of implementation, the molding cavity 52 is arranged as a flute along the plane extruding surface 51 of the molding die 5. The molding cavity 52 can be multi-shape, such as polygon, roundness and other anomalistic shape. The extruding head 1 can be driven by a power drive for linear movement, and molding die can be a static design. A relative slipping movement mode will occur between its slope end surface 41 and the extruding surface 51 of the molding die 5, which forms a relative movement due to differential speed between the extruding head 4 and the extruding surface 51 of the molding die 5. During the plane slipping movement due to differential speed, the bio-substance material particles extruded will be ground, twisted, and flaked. At the same time, along with the continual lessening of the volume for the wedged extruding cavity 3, bio-substance material will finally be extruded into the molding cavity 52 of the molding die 5.

Because the molding cavity 52 is a flute arranged on the extruding surface, bio-substance material in the extruding cavity 3 is stretched to become flake material, which is extruded into the flute of the molding cavity 52. The material, now in the form of a sandwich, will then be pressed into the molding cavity 52 by the small end of the extruding cavity 3. The molding feature in this example of implementation is that because the molding cavity is a flute where the front end is a hard flute wall made from rigid material, the bio-substance material which is extruded into the flute of the molding cavity will be impacted within its force transfer distance. Therefore not only will the molded product have the same structural mold as the above mentioned examples, but its density will be much more uniform and stable. In effect, the molding method and molding mechanism in this example of implementation will be much more suitable for the processing of board material and other available moldable material. The shape of the molding cavity 52 in this example of implementation can be designed according to the required shape of the product.

In this example of implementation, the extruding head 4 can be driven by the power drive to create a linear movement relative to the molding die 5. The molding die is a static design so as to form a movement mode caused from differential speed. Also, the molding die 5 can be driven by power drive to create a linear movement relative to the extruding head 4, where the direction of the movement is from the large end of the extruding cavity 3 to the small end. The extruding head is a static design.

In this example of implementation, the extruding head 4 and molding die 5 can move simultaneously, as shown in Figure 7. The direction of their movement can be either contrary or same, where N_{extruding} ≠ N_{molding}, which forms a relative movement caused from differential speed. The molding effect will be better when N_{extruding} > N_{molding}.

### Example 5

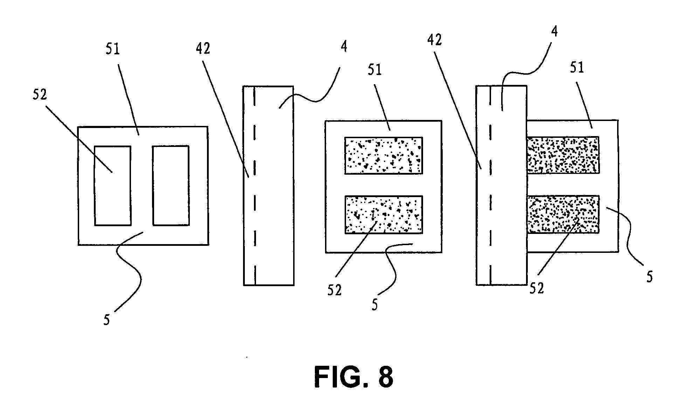

As shown in Figure 8, the molding option and above mentioned movement mode of this example of implementation can arrange two or greater extruding heads 4. There is a slope surface 42 to be arranged at these two extruding heads. An extruding cavity is formed between this slope surface 42 and the end surface 51 of the molding die. During operation, the molding die 5 will extrude the bio-substance material through the previous extruding head 4, which will be pressed and stretched in the cavity 51 arranged at the end surface 51. Henceforth, the molding die will turn at an angle to extrude another layer of material into molding cavity 52 through the next extruding head 4. Because the layer-built flake material in the folding state in the extruding cavity enters into molding cavity 52 at a different angle, every two layers of molding structure will be in a state of intertexture.

In addition, the previous layer of material which enters into the molding cavity 52 will be extruded by the latter layer of material for further extruding. This highly increases its density and causes part of the post-deformation bio-substance particles to enter the gap of another part of the flake particles to further mesh to each other up and down. Consequently, it gives the product a special intertexture structural model, which will have better mechanical features than products produced by existing molding technology. Tests have proven that there is an excellent linking strength for the molding board and reutilized moldable material by using this method, therefore, the molding product produced by using the method described in this example of implementation has excellent linking strength as well. This can minimize the use of any chemical binder, or even eliminate the need to use any binder in furniture fabrication, board or profiled material for decoration, or block-shaped combustible material.

### Example 6

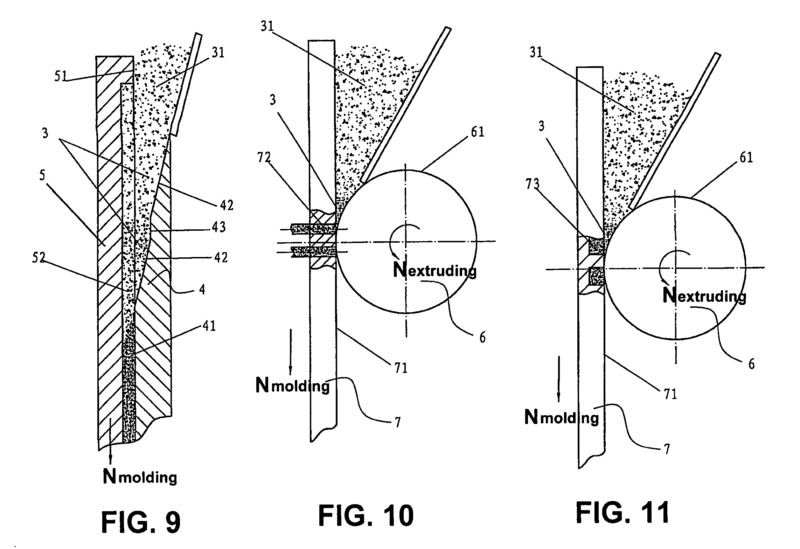

The method and the structure principle of the molding mechanism in this example of implementation are the same as that described in the example of implementation 5. As shown in Figure 9, the extruding head 4 in this example of implementation is a board configuration. This extruding head 4 has an end surface 41 with board configuration, which forms two approximate linear slope surfaces 42. A plane space 43 is arranged between these two slope surfaces 42. The plane space 43 is higher than the end surface 41, which forms the extruding head with ladder configuration. Here, the molding die 5 has a plane extruding surface 51. The arrangement of the end surface 41 of the extruding head 4 is relative to the end surface 51 of the molding die 5, which can form a wedged extruding cavity 3 between the approximate linear slope surface 42 of the extruding head 4 and the extruding surface 51 of the molding die 5. The large end of this extruding cavity 3 is higher than small end. This large end will be connected directly with material inlet 31, and incompact material can enter into wedged extruding cavity 3 from the inlet 31.

The movement mode in the above example of implementation can be used for this example of implementation. The molding principle in this example of implementation is that bio-substance material is ground, twisted, and stretched to be a layer-built flake material in folding state by the action of a shearing force, which will be extruded into the flute of the molding cavity 52. There it will be pressed by the plane space 43 to be a molded product. When the extruding head 4 moves continuously relative to the molding die 5, the slope surface 42 of the plane space 43 will continue to extrude the bio-substance material in the molding cavity 52. During the latter extruding process, not only will the density of the bio-substance get bigger, but also part of the deformed flake particles will enter the gap between the other part of the flake particles to be further meshed to each other up and down. Consequently, it gives the product a special intertexture structural model, which will have better mechanical features than products produced by existing molding technology.

This example of implementation can be used for making usable moldable material, which has higher thickness and intertexture structural model.

### Example 7

For this example of implementation, before molding process, the feedstock of the bio-substance should be in an incompact state.

In this example of implementation, the extruding head is made up of a rolling body. The wedged extruding cavity is formed between the rolling surface of the extruding head and the extruding surface of the molding die, which forms a rolling extruding mode.

The molding principle and the structural principle are shown in Figure 10. The extruding head 6 is a circular column which can turn around its center of rotation, where the circumferential surface forms the rolling surface 61. The extruding surface 71 of the molding die 7 is a plane design. The rolling surface 61 of the extruding head 6 is arranged relative to the extruding surface 71 of the molding die 7, and a wedged extruding cavity is formed between the extruding head 6 and the extruding surface 71 of the molding die 7. The coordination clearance between the rolling surface 61 of the extruding head 6 and the extruding surface 71 of the molding die 7 will be less than 3mm, where the best is a coordination one with no clearance.

Relative motion is caused by differential speed between the rolling surface 61 of the extruding head 6 and the extruding surface 71 of the molding die 7. When it occurs, the material, which enters into the extruding cavity 3 will be applied to a shearing force. The material is ground, stretched, and flaked by the action of the shearing force. The action of the shearing force will produce heat to increase the temperature of the material to 70-110°C. At the same time, the material is extruded to the small end of wedged extruding head 3 and then into the molding cavity 72 of the molding die 7.

There is a molding cavity 72 which is arranged at an angle to the extruding end surface 21 of the molding die 7 for the processing of bio-substance combustion material. The molding cavity 72 can be a column-shaped cavity, which is arranged along the extruding surface 71 so that multi-claviform combustion material can be molded every time. The raw material in the extruding cavity 3 will continuously enter the molding cavity 72 to be molded to a claviform body by the action of shearing force. Previous bio-substance material that enters into molding cavity 72 will be extruded by the latter bio-substance material for further extruding, which will increase its density, and part of bio-substance particles which have been in the extruding cavity 3 to be ground, twisted, and flaked will be deformed. After deformation, the material will enter the gap with another part of flake particles to further mesh to each other up and down. Consequently, it gives the product a special structural model , which will have better mechanical features than that of the product produced by existing molding technology. Finally, the molded combustion material will be extruded from the outlet of the molding cavity 72.

In this example of implementation, the extruding head 6 can be driven by a power drive, and the molding die 7 can be a static design. A relative rolling movement mode will be produced between the rolling surface 61 of the extruding head 6 and the extruding surface 71 of the molding die 7. It can also move in the direction as shown in Figure 10, in which the extruding head 6 rotate along its vertical axis from the large end to the small end, thus creating a relative movement caused by differential speed between the extruding head 6 and the extruding surface 71 of the molding die 7. During the differential speed rolling movement, the bio-substance particles which are extruded in extruding cavity will be ground, twisted, stretched, and flaked. At the same time, the movement direction will force the material to be extruded to the small end of wedged extruding cavity 3 and finally extruded into the molding cavity 72 for molding.

In this example of implementation, when the coordination between the rolling surface 61 of the extruding head 6 and the extruding surface 71 of the molding die 7 presents no clearance, the bio-substance material which has been ground, twisted, and flaked in the extruding cavity 3 will discontinuously (not continuously) enter into the molding cavity 72. In this way, the flake material will be extruded into the molding cavity 72 by layers. Experiments have proven that this method will be much more propitious to overcome the objection of incompact bio-substance material which has lesser force transfer distance so as to make the structural model of bio-substance material much more reasonable after it is molded in the molding cavity 72.

### Example 8

The working principle and principle of structure in this example of implementation are the same as that of the example of implementation 7 so unnecessary details will be omitted. The only difference between this example of implementation and the example of implementation 7 is that the molding die 7 in this example of implementation is a linear move driven by a power drive.

The extruding head 6 in this example of implementation can still turn around its center of rotation driven by a power and the molding die 7 is a linear movement driven by power. Because of this, a relative rolling movement mode will be produced due to differential speed between the rolling surface 61 of the extruding head 6 and the extruding surface 71 of the molding die 7. Because this relative movement is a compositive movement produced by self rotation of the extruding head 6 along its axis of rotation and linear movement of the molding die 7, the linear speed of two extruding surfaces for extruding bio-substance material differs, which forms a shearing force applied to the bio-substance material extruded in between.

In this example of implementation, the direction of self-rotation for the rolling surface 61 of the extruding head 6 is contrary to the direction of the movement for the molding die 7, which makes two extruding surfaces of wedged extruding cavity have the linear speed with contrary direction.

Another implementation mode in this example of implementation is that the direction of self rotation for the rolling surface 61 of the extruding head 6 is the same as the direction of the movement for the molding die 7; namely, two extruding surfaces of the wedged extruding cavity have the linear speed with same direction but different linear speed. In this example, N_{extruding} ≠ N_{molding} it forms a relative rolling movement mode due to the differential speed. The molding effect will be even better when N_{extruding} > N_{molding}.

### Example 9

The working principle and structural principle in this example of implementation are the same as that in the example of implementation 7, therefore, unnecessary details will be omitted. The difference between this example and example of implementation 7 is that the molding cavity 73 of the molding die 7 is arranged in a flute on the extruding surface 71 of the molding die 7.

As shown in Figure 11, in this example of implementation, the molding cavity 73 is a flute arranged along the surface of the plane extruding surface 71 of the molding die 7. A relative movement due to differential rolling speed will occur between the rolling surface 61 of the extruding head 6 and the extruding surface 71 of the molding die 7, which will cause the extruded bio-substance material to be ground, twisted, stretched, and flaked. During this process, as the wedged extruding cavity is getting smaller, the bio-substance material will finally be extruded into the flute of the molding cavity 73 for the molding die 7.

Because the molding cavity 73 is arranged in the flute of the extruding surface 71, the bio-substance material which has been stretched into a flake material will be extruded as a floc into the flute of the molding cavity 73 and pressed from the small end of the extruding cavity 3 in the molding cavity 73 to become a layered structure. The molding feature in this example of implementation is due to the fact that the molding cavity is a flute, and the front end of the flute is a hard wall made from rigid material where the material will be extruded.

Therefore, the bio-substance material which will be extruded into molding cavity 73 will be pressed within its force transfer distance so that not only will only the molded product have the same structural model as in the above mentioned examples of implementation, but also its density will be much more uniform and it will be much stronger. Therefore, the molding method and molding mechanism in this example of implementation are much more suitable for board material and processing other available material. The shape of the molding cavity 73 in this example of implementation can be designed according to the shape of molded products.

The movement mode in this example of implementation can be the same as the compositive movement mode in the example of implementation 8. Based on the movement mode in this example of implementation, two or greater extruded heads 6 can be arranged. When the molding die 7 passes the previous extruding head 6 and one more layer of bio-substance material is extruded into molding cavity 73, it will turn at an angle to pass the next extruding head 6. Then one more layer of bio-substance material will be extruded into molding cavity 73. Because the bio-substance material, which has been stretched into a flake in the extruding cavity, enters into molding cavity 73 from different angles, every two layers of molded structure model will be in the state of intertexture.

In addition, the previous layer of bio-substance material which enters molding cavity 73 will be extruded by the latter layer of material. This highly increases its density and causes part of the post-deformation bio-substance particles to enter the gap of another part of the flake particles to further mesh to each other up and down. Consequently, it gives the product a special intertexture structural model, which will have better mechanical features than products produced by existing molding technology. Tests have proven that there is an excellent linking strength for the molding board and reutilized moldable material by using this method, therefore, the molding product produced by using the method described in this example of implementation has excellent linking strength as well. This can minimize the use of any chemical binder, or even eliminate the need to use any binder in furniture fabrication, board or profiled material for decoration, or block-shaped combustible material.

### Example 10

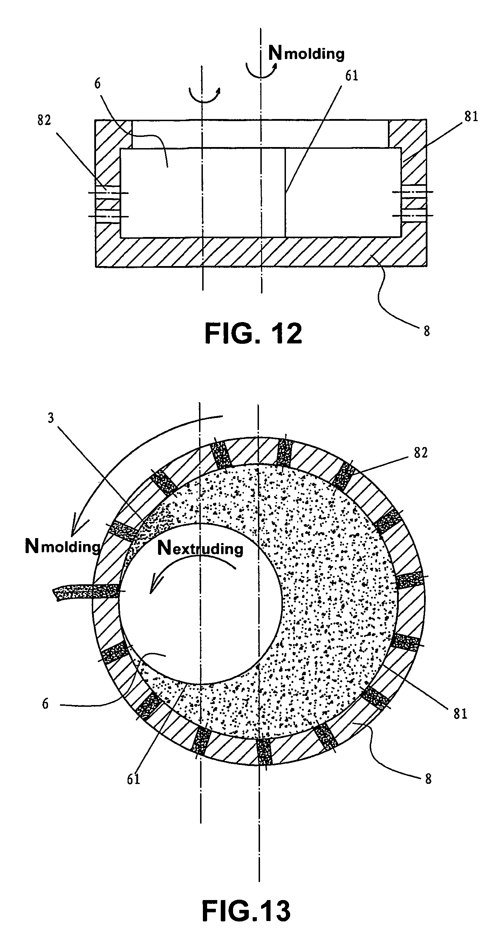

The molding principle and structural principle of the molding mechanism in this example of implementation is shown in Figure12, 13 and 14.

The extruding head 6 in this example of implementation is a circular column. This extruding head 6 can rotate around its center of rotation and is power driven. The circumferential surface forms a rolling surface 61. The molding die 8 can be made from a canister body, where its inner surface forms a circular extruding surface 81 and the curvature of extruding surface 81 is greater than that of rolling surface 61 of the extruding head. When the rolling surface 61 of the extruding head is arranged relative to the extruding surface 81 of the molding die 8, these two curvilinear surfaces between the extruding head 6 and the extruding surface 81 of the molding die 8 will form a wedged extruding cavity 3.

The coordination clearance between the rolling surface 61 of the extruding head 6 and the extruding surface 81 of the molding die 8 is less than 3mm, where the best coordination is no clearance.

When the rolling surface 61 of the extruding head 6 moves relative to the extruding surface 81 of the molding die 8 due to differential speed, the material which enters into extruding cavity 3 is applied to a shearing force during the relative movement, which is ground, twisted, and flaked by the action of shearing force. The friction of the material in extruding cavity 3 will produce heat by the action of the shearing force, which will increase its temperature to 70-110oC. At the same time, the material is extruded to the small end of the wedged extruding cavity 3 and then into the molding cavity 82 of the molding die 8.

There is a molding cavity 82 which is arranged at an angle to the extruding end surface 81 of the molding die 8. For the processing of bio-substance combustion material, this molding cavity 82 can be a columned cavity, which is arranged in the molding die 8 along the extruding surface. Thus it can mold multi-claviform combustion material. The material in the extruding cavity 3 will continuously enter the molding cavity 82 by the action of shearing force to be molded as a claviform body. Previous material in the molding cavity will be continuously extruded by the extruding action of latter entering material, which will make the density of the material increase. At this point, part of the bio-substance particles which have been grinded, twisted, and flaked in the extruding cavity will be deformed. This deformed part of the bio-substance material will enter the gap between the other part of the flake particles to mesh together up and down. This forms a special structural model of the molded product of this invention, which has better mechanical features than that made from existing molding technology. Finally, the molded product of combustion material will be extruded from the outlet end of the molding cavity 82.

The extruding head 6 in this example of implementation can be power driven which will turn around its vertical axes of rotation. The molding die 8 is a static design, thus a relative rolling movement mode between the rolling surface 61 of the extruding head 6 and the extruding surface 81 of the molding die 8 will occur, which forms a relative movement due to the differential speed between the extruding head 6 and the extruding surface 81 of the molding die 8. During the rolling movement due to differential speed, the bio-substance particles, which are extruded in the extruding cavity, will be ground, twisted, stretched, and flaked. At the same time, the movement direction will force the material to be extruded to the small end of the wedged extruding cavity 3, and finally into the molding cavity 82 of the molding die.

In this example of implementation, if there is no clearance for the coordination between the rolling surface 61 of the extruding head 6 and the extruding surface 81 of the molding die 8, the bio-substance material in the flake state which has been ground, twisted, and flaked in the extruding cavity 3 will discontinuously (not continuously) enter into the molding cavity 82. Thus, the flake material in the molding cavity 82 will be pressed into the molding cavity by layers. Experiments have proven that this method will be much more propitious to overcome the objection of incompact bio-substance material which has lesser force transfer distance, so as to make the structural model of bio-substance material much better after it is molded in the molding cavity 82.

In this example of implementation, the rotating direction of self-rotation for the rolling surface 61 of the extruding head 6 can be the same as that of the molding die 8, which makes the two extruding surfaces of the wedged extruding cavity have linear speed with contrary direction, as shown in Figure 12 and 13.

Another implementation of this example is that the rotating direction of self-rotation for the rolling surface 61 of the extruding head 6 is contrary to that of the molding die 8. Namely, the linear speed of the two extruding surfaces for the wedged extruding cavity have the same direction but with different linear speed, that is, N_{extruding} ≠ N_{molding}. Consequently, this forms a rolling relative movement due to differential speed. The molding effect will be better when N_{extruding} > N_{molding} .

The molding principle in this example of implementation is the same as that in the example of implementation 7.

### Example 11

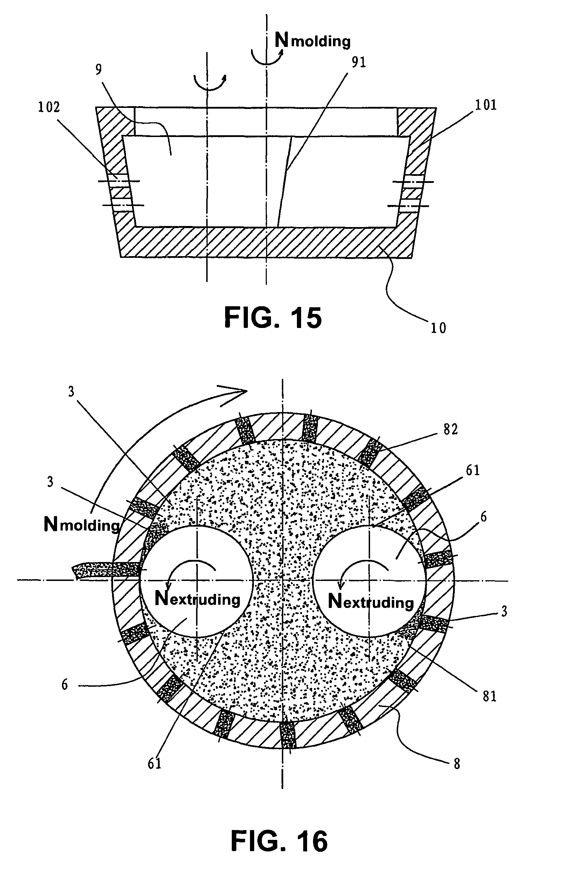

The molding principle and structure principle of this example of implementation are as shown in Figure 15.

The extruding head 9 in this example of implementation is a cone, which can rotate around its center of rotation and is power driven. The circumferential surface forms a rolling surface 91. The molding die 10 can be made from a conic body, where its inner surface forms a conic extruding surface 101 and the curvature of the extruding surface 101 is greater than that of the rolling surface 91 of the extruding head. When the rolling surface 91 of the extruding head 9 is arranged relative to the extruding surface 101 of the molding die 10, these two curvilinear surfaces between the extruding head 9 and the extruding surface 101 of the molding die 10 will form a wedged extruding cavity 3.

The extruding head 9 in this example of implementation can be power driven, which will rotate surrounding its vertical axis of rotation. The molding die 10 is a static design, thus, a relative rolling movement mode between the rolling surface 91 of the extruding head 9 and the extruding surface 101 of the molding die 10 will occur. Also, the extruding head 9 and the molding die 10 will rotate along self axes, respectively, with the same or contrary direction. When the rotations are in the same direction, N_{extruding} ≠ N_{molding}, it forms a relative movement due to the differential speed between the extruding head 9 and the extruding surface 101 of the molding die 10.

The molding principle in this example of implementation is the same as that in the example of implementation 10, and therefore, unnecessary details will be omitted.

### Example 12

The molding principle and structural principle in this example of implementation are as shown in Figure 16.

The extruding head 6 in this example of implementation is a circular column. This extruding head 6 can rotate around its center of rotation and is power driven. The circumferential surface forms a rolling surface 61. The molding die 8 can be made from a canister body, where its inner surface forms a circular extruding surface 81 and the curvature of the extruding surface 81 is greater than that of the rolling surface 61 of the extruding head. When the rolling surface 61 of the extruding head is arranged relative to the extruding surface 81 of the molding die 8, these two curvilinear surfaces between the extruding head 6 and the extruding surface 81 of the molding die 8 will form a wedged extruding cavity 3.

In this example of implementation, more than two extruding heads 6 can be arranged, where every extruding head is power driven and will self-rotate along its vertical axis. The molding die 8 is a static design and the rolling surface 61 of the multi-extruding head 6 will form more than two wedged extruding cavities 3 relative to the extruding surface 81 of the molding die 8.

The movement mode in this example of implementation can be the same and the molding principle is the same as that in the example of implementation 10. Therefore, unnecessary details will be omitted.

The movement mode, as shown in Figure 16, can be adopted also for this example of implementation where the extruding head 6 will self-rotate along its axis of rotation, and the rotation of the molding die 8 is power driven. The relative movement between the extruding head and the molding die is a compositive movement from the self-rotation of the extruding head along its axis of rotation and the revolution of the extruding head relative to the molding die as the center of rotation.

During the operation, when there is no clearance for the coordination between the rolling surface 61 of the extruding head 6 and the extruding surface 81 of the molding die 8, the bio-substance material in the flake state, which has been ground, twisted, and flaked in the extruding cavity 3, will discontinuously (not continuously) enter into the molding cavity 82. Thus, the flake material in the molding cavity 82 will be pressed into the molding cavity by layers. Experiments have proven that this method will be much more propitious to overcome the objection of incompact bio-substance material which has lesser force transfer distance so as to make the structure model of bio-substance material much better after it is molded in the molding cavity 82.

The molding principle in this example of implementation and its effects are the same as that in the above mentioned example of implementation, therefore unnecessary details will be omitted.

### Example 13

The molding principle and structural principle in this example of implementation are as shown in Figure 17.

The extruding head 6 in this example of implementation is a circular column. This extruding head 6 can rotate around its center of rotation and is power driven. The circumferential surface forms a rolling surface 61. The molding die 8 can be made from a canister body, where its inner surface forms a circular extruding surface 81 and the curvature of extruding surface 81 is greater than that of the rolling surface 61 of the extruding head. When the rolling surface 61 of the extruding head is arranged relative to the extruding surface 81 of the molding die 8, the two curvilinear surfaces between the extruding head 6 and the extruding surface 81 of the molding die 8 will form a wedged extruding cavity 3.

In this example of implementation, more than two extruding heads 6 can be arranged and every extruding head is power driven and will self-rotate along its vertical axis. The molding die 8 is a static design and the rolling surface 61 of the multi-extruding head 6 will form more than two wedged extruding cavities 3 relative to the extruding surface 81 of the molding die 8.

The movement mode in this example of implementation has the direction of self-rotation for the extruding head 6 along its axis of rotation is spinning contrary to that of the revolution of the extruding head relative to the molding die as the center of rotation. The self-rotation of the extruding head 6 along its axis of rotation and the revolution of the extruding head 6 relative to the molding die 8 as the center of rotation can be a movement of differential speed but in the same direction. The linear speed of self-rotation for the extruding head 6 along its axis of rotation is greater than the revolution speed of the extruding head 6 relative to the molding die 8.

During the operation, when there is no clearance for the coordination between the rolling surface 61 of the extruding head 6 and the extruding surface 81 of the molding die 8, the bio-substance material in the flake state which has been ground, twisted, and flaked in the extruding cavity 3 will discontinuously (not continuously) enter into the molding cavity 82. Because there is a differential speed of self-rotation between the extruding head and the molding die, the flake material in the molding cavity 82 will be pressed into the molding cavity 82 by layers. Experiments have proven that this method will be much more propitious to overcome the objection of incompact bio-substance material which has lesser force transfer distance so as to make the structure model of bio-substance material much better after it is molded in the molding cavity 82.

The molding principle in this example of implementation and its effects are the same as that in the above mentioned example of implementation, therefore, unnecessary details will be omitted.

## Claims

1. A molding method for moldable bio-substance material, comprising:
(1) crushing the bio-substance materials into an incompact state and then extruding them for further molding through a molding die;
(2) forming at least a wedged extruding cavity between an extruding head and an extruding surface of the molding die;
(3) passing the bio-substance materials in the incompact state through an action of a relative movement between the extruding head and the extruding surface of the molding die at differential speeds; and
(4) grinding the particles of the bio-substance materials extruded between the extruding head and the extruding surface, and then twisting, stretching and flaking said bio-substance materials, and meanwhile extruding the same to a small end of the wedged extruding cavity and further forcing them into a molding cavity of the molding die to be molded.

2. The molding method for moldable bio-substance material according to claim 1, wherein the water content of said moldable bio-substance materials is higher than 6% in its extruding state, and the best is 20%~50% for extrusion.

3. The molding method for moldable bio-substance material according to claim 1, wherein said wedged extruding cavity can be formed between an end surface of the extruding head and the extruding surface of the molding die, and said bio-substance materials enter the extruding cavity from a large end of said wedged extruding cavity.

4. The molding method for moldable bio-substance material according to claim 3, wherein the end surface of the extruding head forms at least a slope surface; the wedged extruding cavity is formed between said slope surface and the extruding surface of the molding die; the extruding cavity is arranged along the opposite direction of the movement of the extruding head from the large end to a small end thereof; and said bio-substance materials are grinded, twisted up, and extruded to the small end of the wedged extruding cavity and meanwhile extruded into the molding cavity when the extruding head moves relatively to the extruding surface of the molding die.

5. The molding method for moldable bio-substance material according to claim 4, wherein said end surface of the extruding head and the extruding surface of the molding die have, in between, a coordinative clearance of less than 3mm.

6. The molding method for moldable bio-substance material according to claim 5, wherein said end surface of the extruding head and the extruding surface of the molding die do not have a coordinative clearance in between.

7. The molding method for moldable bio-substance material according to claim 3 or 4, wherein said relative movement of the extruding head and the extruding surface of the molding die is a relative slipping movement.

8. The molding method for moldable bio-substance material according to claim 7, wherein during said relative movement between the extruding head and the extruding surface of the molding die, the extruding head can turn along its vertical axes while the molding die is static.

9. The molding method for moldable bio-substance material according to claim 3 or 4, wherein said relative movement between the extruding head and the extruding surface of the molding die is a relative parallel movement in between.

10. The molding method for moldable bio-substance material according to claim 9, wherein during said relative movement one of the extruding head and the molding die us still, while the other is moving relatively parallel to the static one.

11. The molding method for moldable bio-substance material according to claim 9, wherein said parallel movement between the extruding head and the molding die is caused by their opposite movements.

12. The molding method for moldable bio-substance material according to claim 9, wherein said parallel movement between the extruding head and the molding die is caused by their differential speed moving in the same direction.

13. The molding method for moldable bio-substance material according to claim 3, wherein said molding cavity is arranged at an angle toward the end surface of the molding die.

14. The molding method for moldable bio-substance material according to claim 13, wherein said molding cavity arranged at an angle to the end surface of the molding die has an oriented section.

15. The molding method for moldable bio-substance material according to claim 9, wherein said molding cavity is a flute distributed along the extruding surface of the molding die.

16. The molding method for moldable bio-substance material according to claim 1, wherein said extruding head is made from a rolling body, and the wedged extruding cavity is formed between the rolling surface of the extruding head and the extruding surface of the molding die.

17. The molding method for moldable bio-substance material according to 16, wherein said extruding head made from the rolling body is a cylinder.

18. The molding method for moldable bio-substance material according to 16, wherein said extruding head made from the rolling body is conic.

19. The molding method for moldable bio-substance material according to claim 16 or 17 or 18, wherein said relative movement between the extruding head and the molding die is a composite movement formed between an axial self-rotation of the extruding head and a linear movement of the molding die.

20. The molding method for moldable bio-substance material according to claim 16 or 17 or 18, wherein one of the extruding head and the molding die is static, while the other moves relatively to the static one.

21. The molding method for moldable bio-substance material according to claim 19, wherein the direction of said axial self-rotation of the extruding head is opposite to the direction of the linear movement of the molding die.

22. The molding method for moldable bio-substance material according to claim 21, wherein said relative movement is a differential speed movement in the same direction of the axial self-rotation of the extruding head and the linear movement of the molding die.

23. The molding method for moldable bio-substance material according to claim 22, wherein the linear speed of said axial self-rotation of the extruding head is larger than the linear moving speed of the molding die.

24. The molding method for moldable bio-substance material according to claim 16 or 17 or 18, wherein said relative movement between the extruding head and the molding die is composed of an axial self-rotation of the extruding head and a revolution of the extruding head relatively using the molding die as an axe of the rotation.

25. The molding method for moldable bio-substance material according to claim 24, wherein the relative movement of the extruding head and the molding die is composed of the axial self-rotation of the extruding head and the revolution of the extruding head relative to the molding die as an axe of rotation.

26. The molding method for moldable bio-substance material according to claim 24, wherein said relative movement of the extruding head to the molding die is composed of the axial self-rotation of the extruding head and an axial self-rotation of the molding die.

27. The molding method for moldable bio-substance material according to claim 25, wherein the movements of the extruding head and the molding die are in opposite direction between the axial self-rotation of the extruding head and the revolution of the extruding head using the molding die as a rotation axe.

28. The molding method for moldable bio-substance material according to claim 25, wherein the relative movement is a differential speed movement in the same direction between the said axial self-rotation of the extruding head and the revolution of the extruding head using molding die as a rotation axe.

29. The molding method for moldable bio-substance material according to claim 28, wherein the linear speed of the axial self-rotation of the extruding head is greater than the revolution speed of the extruding head using the molding die as the rotation axe.

30. The molding method for moldable bio-substance material according to claim 19, wherein the extruding surface of the molding die is a plane; said wedged extruding cavity is formed between the rolling surface of the extruding head and the extruding surface of the molding die, and the materials enter into the extruding cavity along the large end of the wedged extruding cavity.

31. The molding method for moldable bio-substance material according to claim 24, wherein said extruding surface of the molding die is an arc, and the curvature radius of the extruding arc surface is greater than the rotation radius of the extruding head; and said wedged extruding cavity is formed between the rolling surfaces of the extruding head and the extruding surface of the molding die, and the bio-substance materials enter into the extruding cavity along the large end of the wedged extruding cavity.

32. The molding method for moldable bio-substance material according to claim 30 or 31, wherein a large end of the wedged extruding cavity has an opening facing upwards, and the bio-substance materials are extruded into the extruding cavity and moved to a small end by gravity and friction produced between the extruding head and the extruding surface.

33. The molding method for moldable bio-substance material according to claim 16 or 17 or 18, wherein said molding cavity of the molding die is arranged at an angle relatively to the extruding surface of the molding die.

34. The molding method for moldable bio-substance material according to claim 33, wherein an oriented section is set for the molding cavity which is arranged at an angle to the extruding surface of the molding die.

35. The molding method for moldable bio-substance material according to any of claims 16 to 18, wherein said molding cavity of the molding die is distributed over multiple flutes on the extruding surface of the molding die.

36. , The molding method for moldable bio-substance material according to any claim above, wherein the said molded product can be either air dried or stove dried.

37. A molding apparatus for moldable bio-substance material, comprising
(1) at least an extruding head driven by power;
(2) a molding die having an extruding surface and a molding cavity;
(3) a wedged extruding cavity formed at least between the extruding head and the extruding surface of the molding die, and having a large end and a small end; and
(4) a material inlet provided at the large end of the wedged extruding cavity, through which particles of the bio-substance materials enter into the extruding cavity to be grinded, twisted up, stretched, flaked due to an action of relative movement between the extruding head and the extruding surface of the molding die, and meanwhile to be molded in the molding cavity of the molding die.

38. The molding apparatus according to claim 37, wherein said wedged extruding cavity is formed between the end surface of the extruding head and the extruding surface of the molding die, and the bio-substance material enter the extruding cavity from the large end.

39. The molding apparatus according to claim 38, wherein at least one slope surface is formed on the end surface of extruding head; the wedged extruding cavity is formed by this slope surface and the extruding surface of the molding die; the extruding cavity is arranged along an opposite direction of the large end to small end movement of the extruding head; and the material will be grinded, twisted up, and at the same time extruded to the small end of extruding cavity and then into molding cavity, when the extruding head moves relatively to the extruding surface of the molding die.

40. The molding apparatus for moldable bio-substance material according to claim 39, wherein the slope surface of the said end surface of the extruding head can be two or more than two, the best to be a uniform distribution.

41. The molding apparatus for moldable bio-substance material according to claim 39, wherein the coordination clearance between the said end surface of the extruding head and the said extruding surface of the molding die is less than 3mm.

42. The molding apparatus for moldable bio-substance material according to claim 41, wherein there is no coordination clearance between the said end surface of the extruding head and the said extruding surface of the molding die.

43. The molding apparatus for moldable bio-substance material according to claim 39 or 40, wherein the said relative movement between the end surface of the extruding head and the extruding surface of the molding die can be a relative slipping movement.

44. The molding apparatus for moldable bio-substance material according to claim 43, wherein the said relative movement includes an axial rotation of the extruding head and the static state of the molding die.

45. The molding apparatus for moldable bio-substance material according to claim 40 or 44, wherein the said slope surface of the end surface of the extruding head can be annularly distributed with the axe of its rotation.

46. The molding apparatus for moldable bio-substance material according to claim 39, wherein the said relative movement between the extruding head and the molding die can be relatively parallel.

47. The molding apparatus for moldable bio-substance material according to claim 46, wherein for the said extruding head and said molding die, one of which is static and the other one is moving parallel relative to the static one.

48. The molding apparatus for moldable bio-substance material according to claim 46, wherein the said relatively parallel movement between the extruding head and the molding die is the contrary movement in between.

49. The molding apparatus for moldable bio-substance material according to claim 46, wherein the said relatively parallel movement between the extruding head and the molding die is the differential speed movement in the same direction.

50. The molding apparatus for moldable bio-substance material according to claim 46, wherein the said slope surface of the end surface for the extruding head can be vertically distributed according to the direction of its movement.

51. The molding apparatus for moldable bio-substance material according to claim 39, wherein the said the end surface of the molding cavity is set at an angle to the molding die.

52. The molding apparatus for moldable bio-substance material according to claim 51, wherein an oriented section could be set in the said the extruding cavity, which is arranged at an angle to the extruding surface of the molding die.

53. The molding apparatus for moldable bio-substance material according to claim 51, wherein the large end height of the said molding cavity oriented section is no greater than 10mm.

54. The molding apparatus for moldable bio-substance material according to claim 39 or 46, wherein the said molding cavity can be composed of multiple flutes distributing along the extruding surface of the molding die.

55. The molding apparatus for moldable bio-substance material according to claim 37, wherein the said extruding head can be composed of the rolling body; the said wedged extruding cavity is formed between the rolling surface of the extruding head and the extruding surface of the molding die.

56. The molding apparatus for moldable bio-substance material according to claim 55, wherein the said rolling body made of the extruding head, can be a cylinder.

57. The molding apparatus for moldable bio-substance material according to claim 55, wherein the said rolling body made of the extruding head, can be a cone.

58. The molding apparatus for moldable bio-substance material according to claim 55, wherein the said extruding surface of the molding die is set as a plane; the said wedged extruding cavity is formed between the rolling surface of the extruding head and the extruding surface of the molding die; the material enters the extruding cavity from the large end of the wedged extruding cavity.

59. The molding apparatus for moldable bio-substance material according to claim 55, wherein the said extruding surface of molding die has the shape of an arc; the curvature radius of the extruding arc surface is greater than the rotation radius of the extruding head; the said wedged extruding cavity is formed between rolling surface of the extruding head and extruding surface of the molding die; the material enters the extruding cavity through the large end of wedged extruding cavity.

60. The molding apparatus for moldable bio-substance material according to claim 58 or 59, wherein the said large end of wedged extruding cavity has upward facing opening; the material is extruded to enter the extruding cavity and then to the small end by its gravity and friction generated between the extruding head and the extruding surface.

61. The molding apparatus for moldable bio-substance material according to claim 55, 56 or 57, wherein the said relative movement between the extruding head and the molding die is a compositive movement, which includes the axial self-rotation of the extruding head and the linear movement of the molding die.

62. The molding apparatus for moldable bio-substance material according to claim 55, 56 or 57, wherein for the said extruding head and molding die, one is static, the other has relative movement relative to the static one.

63. The molding apparatus for moldable bio-substance material according to claim 61, wherein the said direction of extruding head axial self-rotation is contrary to the linear movement direction of the molding die.

64. The molding apparatus for moldable bio-substance material according to claim 61, wherein the said relative movement is the differential speed movement in the same direction between the axial self-rotation of the extruding head and linear movement of the molding die.

65. The molding apparatus for moldable bio-substance material according to claim 64, wherein the linear speed of the said extruding head axial self-rotation, is larger than the linear moving speed of the molding die.

66. The molding apparatus for moldable bio-substance material according to claim 55, 56 or 57, wherein the said relative movement between the extruding head and molding die, is a composition movement including the axial self-rotation of the extruding head and the revolution movement of the extruding head using molding die as rotation axle.

67. The molding apparatus for moldable bio-substance material according to claim 66, wherein the said relative movement between the extruding head and the molding die, is a composition movement including the axial self-rotation of the extruding head and the revolution movement of the extruding head using extruding die as rotation axle.

68. The molding apparatus for moldable bio-substance material according to claim 66, wherein the said relative movement between the extruding head and the molding die, is a composition movement including the axial self-rotation of the extruding head and the axial rotation of the molding die.

69. The molding apparatus for moldable bio-substance material according to claim 67 or 68, wherein the said direction of the extruding head axial self-rotation is contrary to that of the revolution movement of extruding head using molding die as rotation axle.

70. The molding apparatus for moldable bio-substance material according to claim 67 or 68, wherein it is the same direction differential speed movement that is between the said axial self-rotation of the extruding head and the revolution movement of the extruding head using molding die as rotation axle.

71. The molding apparatus for moldable bio-substance material according to claim 70, wherein the said linear speed of the extruding head axial self-rotation is greater than the revolution speed of the extruding head relative to the molding die.

72. The molding apparatus for moldable bio-substance material according to any claim from 55 to 71, wherein the said molding cavity of the molding die can be set at an angle relative to the extruding surface of the molding die.

73. The molding apparatus for moldable bio-substance material according to claim 72, wherein an oriented section can be set on the said molding cavity, which is set at an angle to the extruding surface of the molding die.

74. The molding apparatus for moldable bio-substance material according to claim 73, wherein the height of the large end is no greater than 10mm in the said oriented section of the molding cavity.

75. The molding apparatus for moldable bio-substance material according to any claim from 55 to 72, wherein the said molding cavity of the molding die can be distributed among multiple flutes on the extruding surface of the molding die.
